# DEMANDE DE BREVET EUROPEEN

(11) **EP 1 527 760 A1**
(43) Date de publication de la demande: **04.05.2005**
(21) Numéro de dépôt: 03024751.4
(22) Date de dépôt: 29.10.2003
(51) Int. Cl.: A61F 7/02

(54) **Coussin thermique et son utilisation**

(71) Demandeur: Normand, Jacques, 1050 Bruxelles (BE); Jouhannet, Guy, 3724 Vliermaal (BE); Bossut, Philippe, 6970 Tenneville (BE)
(72) Inventeur: Jouhannet, Guy, 3724 Vliermaal (BE)

(57) **Abrégé**

Coussin thermique comprenant un réseau de blocs articulés (1), dont une partie au moins comprend une matière thermique, les blocs formant entre-eux des interstices (8) comblés avec une matière thermique déformable.

## Description

### Domaine de l'invention

L'invention se rapporte aux coussins thermiques, ainsi qu'à leur utilisation.

### Etat de la technique

Le secteur hospitalier et la médecine ambulatoire font un usage important de coussins thermiques, notamment pour soulager la douleur ou dans diverses thérapies telles que la cryothérapie par exemple.

On sait en effet que l'application du froid peut soulager la douleur (maux de tête, migraine, maux de dents, douleurs musculaires ou inflammatoires, etc.) ou favoriser la résorption d'hématomes, d'oedèmes et la cicatrisation de plaies accidentelles ou chirurgicales.

Dans le brevet Etats-Unis US 3 545 230, on propose un coussin réfrigérant qui utilise la chaleur latente de fusion d'une substance solide. Durant cette phase de fusion, la température reste constante. Le coussin comprend une couche d'un gel hydrophile et insoluble, dans une enveloppe étanche et flexible. Un substrat inerte et flexible (par exemple des fibres ou un tissu), noyé dans le gel, sert à renforcer la résistance mécanique du coussin et à lui permettre d'épouser une forme géométrique quelconque. Pour conférer une flexibilité suffisante au coussin, on superpose plusieurs couches de gel de très faible épaisseur (quelques millimètres à peine) et on interpose un film flexible et inerte entre les couches de gels. Ce coussin connu présente le désavantage d'être de construction difficile et coûteuse, ses performances sont faibles et ses applications sont limitées.

Dans le document EP 0 123 949, la substance cryogénique du coussin consiste en des morceaux de gel de constitution particulière, présentant une élasticité comparable à celle du caoutchouc et ne collant pas entre-eux à la température de production du froid. Le déplacement des morceaux de gel les uns par rapport aux autres dans le coussin confère à celui-ci la souplesse recherchée. Ces coussins connus ont toutefois le désagrément de nécessiter un gel coûteux et leur fabrication est délicate et coûteuse.

Dans le brevet Etats-Unis US 3 885 403, on suggère d'utiliser pour la substance cryogénique, un gel contenant une grande proportion d'un agent abaissant le point de congélation au-dessous de la température normale d'utilisation. Pour l'agent abaissant le point de congélation, on propose la glycérine et le propylène glycol. Avec ce coussin connu, la production du froid ne résulte pas d'une chaleur latente de fusion, mais du réchauffement progressif du gel. Ce coussin connu présente la propriété d'être de construction simple et de conserver une bonne flexibilité et une bonne aptitude à la déformation souple, du fait que le gel ne passe pas par une phase solide. Il présente par contre l'inconvénient d'une courte durée d'utilisation, puisque la production du froid ne résulte pas d'une chaleur latente de fusion, mais du réchauffement progressif du gel. En outre, la productivité du cousin (la production de froid ou l'extraction de calories par unité de temps) n'est pas constante mais diminue au fur et à mesure que le gel se réchauffe, ce qui constitue un désavantage supplémentaire de ce coussin connu.

Dans le document WO 97/11657, on décrit des panneaux alvéolaires formés d'un quadrillage de cellules remplies d'un agent thermique. Entre les cellules, le panneau présente des zones rectilignes à faible limite de rupture, qui permettent de le diviser à volonté (par cisaillement ou traction) en coussins thermiques de dimensions prédéterminées en fonction des applications auxquelles ces coussins sont destinés. Dans ces panneaux alvéolaires, les interstices sont la cause de plusieurs désavantages. D'une part, ils réduisent la capacité thermique du panneau et font d'autre part obstacle à une action homogène du panneau, lorsque celui-ci est appliqué sur la peau d'un patient.

### Résumé de l'invention

L'invention vise à remédier aux désavantages énoncés plus haut des coussins thermiques connus et à répondre aux besoins des utilisateurs potentiels (notamment en milieu hospitalier), en fournissant un coussin thermique de conception nouvelle, qui concilie une excellente flexibilité/élasticité, capable de s'adapter parfaitement même sur des formes en mouvement, avec un pouvoir calorifique élevé et durable à température sensiblement constante, dont l'action est homogène (notamment lorsqu'il est utilisé sur une partie du corps humain), dont l'épaisseur peut être maintenue sensiblement constante en cours d'utilisation, dont les dimensions et la forme sont sensiblement illimitées, qui peut subir des variations de pressions importantes sans se déformer, et qui, grâce aux propriétés énoncées plus haut, peut s'adapter à de multiples usages, notamment en milieu hospitalier, sans nécessiter des manipulations importantes.

En conséquence, l'invention concerne un coussin thermique comportant un réseau de blocs dont une partie au moins comprend une matière thermique, ledit coussin se caractérisant en ce que les blocs sont articulés et forment entre eux des interstices qui sont comblés au moins partiellement avec une matière thermique déformable.

Le coussin thermique selon l'invention est destiné à être mis en contact avec un corps matériel, dans le but d'influer sur les échanges de chaleur de ce corps avec le milieu ambiant.

L'expression « corps » doit être prise dans une acceptation générale, désignant un objet matériel. Elle désigne indifféremment un corps solide, un corps liquide ou un corps gazeux. Dans le cas d'un corps solide, celui-ci peut par exemple comprendre la surface d'un objet solide ou une partie de l'anatomie d'un animal ou d'un être humain. Dans le cas d'un corps liquide, celui-ci peut comprendre la surface d'une nappe ou d'un bain liquide. Dans cette application de l'invention, le coussin peut par exemple servir de cloison étanche entre deux liquides distincts ou entre un liquide et une atmosphère environnante, par exemple l'ambiance.
Dans le cas d'un corps gazeux, celui-ci comprend une atmosphère. Dans cette application de l'invention, le coussin selon l'invention peut par exemple former un écran entre deux atmosphères distinctes, par exemple une atmosphère chaude (l'atmosphère d'un four industriel) et une atmosphère à température modérée.
En variante, dans le cas d'un corps liquide ou gazeux, celui-ci peut éventuellement être enfermé dans une enveloppe étanche, par exemple une membrane souple.

Le milieu ambiant peut être un milieu gazeux ou un milieu liquide. Dans le cas d'un milieu gazeux, il peut être par exemple l'air atmosphérique, l'atmosphère d'un local chauffé, l'atmosphère d'un local réfrigéré (par exemple une chambre frigorifique) ou l'atmosphère d'un four industriel (liste non exhaustive). Dans le cas où le milieu ambiant est un milieu liquide, celui-ci peut par exemple être de l'eau ou un bain chimique industriel.

Dans la suite du présent mémoire, on désigne par l'expression « température normale d'utilisation », la température de la matière thermique des blocs et/ou des interstices, au moment de l'utilisation du coussin.

Dans la suite du présent mémoire, le mot « coussin » désigne de manière très générale tout élément solide et souple, destiné à être mis en contact avec le corps (tel que défini plus haut) pour le couvrir au moins partiellement ou l'envelopper totalement ou partiellement. Le coussin thermique selon l'invention peut dès lors être conformé de manière très diverse selon l'usage auquel on le destine. Il peut par exemple avoir la forme d'un objet de literie (oreiller, couverture, matelas), d'une poche, d'un manchon, d'un article vestimentaire ou d'une partie de vêtement, d'un emballage, d'un écran ou rideau, d'un revêtement (liste non exhaustive). Il peut être réalisé en modules divers assemblables.

Le coussin thermique selon l'invention est un coussin qui, lorsqu'il est mis en contact avec un corps (selon la définition donnée plus haut), à la température normale d'utilisation, engendre un transfert de chaleur entre ledit coussin et ledit corps ou concourt à maintenir la température dudit corps sensiblement constante.
Le coussin thermique selon l'invention est désigné « coussin calorifique » ou « coussin cryogénique » lorsque sa température normale d'utilisation est différente de celle du corps avec lequel on le met en contact, de telle sorte qu'un transfert de chaleur s'opère entre ledit coussin et ledit corps. Lorsque le transfert de chaleur s'opère naturellement dans le sens du corps vers le coussin thermique, celui-ci est un coussin cryogénique. Dans le cas où le transfert de chaleur s'opère dans le sens du coussin thermique vers le corps, le coussin thermique selon l'invention est un coussin calorifique.
Le coussin thermique selon l'invention est désigné « coussin isothermique » lorsque sa température normale d'utilisation est sensiblement égale à celle du corps avec lequel on le met en contact, de telle sorte qu'il n'y ait pas de transfert de chaleur entre ledit corps et le coussin thermique. Un coussin isothermique conforme à l'invention exerce dès lors la fonction technique d'un isolant calorifique, en faisant obstacle à un transfert de chaleur entre le corps et le milieu ambiant.

Le coussin thermique selon l'invention comprend un réseau de blocs, entre lesquels sont ménagés des interstices.

Par définition, chaque bloc du coussin est un élément solide. La forme des blocs du coussin selon l'invention sera détaillée plus loin.

Les blocs sont assemblés en réseau. Ils sont par ailleurs espacés de manière à former entre-eux un réseau d'interstices. Le coussin selon l'invention comprend de la sorte un entrelacs d'interstices dans un entrelacs de blocs. Le réseau de blocs comprend généralement une seule couche de blocs, bien qu'un réseau formé de plusieurs couches superposées de blocs ne soit pas exclu de l'invention.

Les blocs du réseau sont articulés entre-eux. En d'autres termes, les blocs du réseau sont reliés entre-eux par des articulations. Des détails concernant les articulations seront fournis plus loin.

Dans le coussin selon l'invention, la forme et les dimensions des blocs, la forme et les dimensions des interstices et le choix des articulations sont adaptés pour que le réseau de blocs forme une structure solide, déformable par flexion et/ou torsion et/ou rotation autour d'axes multiples répartis dans un espace à trois dimensions. Cette particularité du coussin selon l'invention lui permet de s'adapter facilement à pratiquement n'importe quelle forme de corps sur lequel on l'applique (par exemple une partie de l'anatomie humaine ou animale) et d'en accompagner le mouvement.

Dans le coussin selon l'invention, les blocs et les interstices contiennent une matière thermique. La matière thermique sera explicitée plus loin.

La forme des blocs dépend de l'usage du coussin et n'est pas critique pour la définition de l'invention. Les blocs peuvent par exemple avoir une forme sphérique, hémisphérique, ovoïde, annulaire, lenticulaire, conique, tronconique ou polyédrique. Les blocs peuvent avoir des faces planes, cintrées ou gauches, par exemple hélicoïdales ou comprendre un assemblage de faces planes et de faces cintrées ou gauches. Ils peuvent par exemple avoir la forme de tonnelets qui associent une face annulaire cintrée et des faces planes aux extrémités. De manière générale, toute forme convient, qui, associée aux interstices et aux articulations liant les blocs, permet une déformation du coussin comme explicité plus haut.
Les formes polyédriques sont généralement préférées. Parmi les formes polyédriques, on préfère les polyèdres droits ou pyramidaux. Les pyramides tronquées sont spécialement recommandées. Parmi les polyèdres droits ou pyramidaux, on préfère les polyèdres à base triangulaire, carrée, rectangulaire ou octogonale.

Dans une forme de réalisation préférée de l'invention, chaque bloc est formé de deux troncs de pyramide joints le long de leur grande base, par exemple deux troncs de pyramide triangulaire, carrée ou octogonale. Dans cette forme de réalisation préférée de l'invention, les articulations des blocs sont avantageusement disposées dans le plan géométrique de la grande base susdite des deux troncs de pyramide.

Comme exposé plus haut, les interstices ménagés entre les blocs ont pour fonction de permettre un déplacement de ces blocs autour des articulations qui les lient. La forme des interstices va dépendre de divers paramètres, tels que le type de déformations souhaitées pour le réseau de blocs (flexion uniaxiale, biaxiale ou triaxiale ; torsion ; extension ; ou combinaison de deux ou plusieurs de ces types de déformations), l'amplitude des déformations, la forme et les dimensions des blocs, les articulations utilisées et la disposition de ces articulations entre les blocs.

Le choix des articulations liant les blocs va également être déterminé par le type de déformations souhaitées pour le réseau de blocs. Tout type d'articulation compatible avec la déformation recherchée peut convenir. Des exemples d'articulations utilisables dans le coussin thermique selon l'invention comprennent notamment des charnières métalliques ou en matière plastique, des languettes ou des membranes flexibles, et des pivots. Les articulations peuvent posséder des propriétés élastiques ou être exemptes de telles propriétés.

En variante, en plus des articulations, les blocs du réseau peuvent être reliés entre-eux par des tenons rigides, dont la résistance mécanique à la flexion est faible et contrôlée. Dans cette variante de l'invention, les tenons rigides servent à conférer une rigidité au coussin pour faciliter sa manutention et elles se brisent dès que l'on applique le coussin sur le corps et le déforme.

Conformément à l'invention, les blocs comprennent une matière thermique. La matière thermique des blocs confère ses propriétés thermiques au coussin thermique selon l'invention.
Dans le cas d'un coussin cryogénique, la matière thermique des blocs est sélectionnée parmi celles qui, portées à la température normale d'utilisation et soumises ensuite à un refroidissement, libèrent une quantité de chaleur importante.
Dans le cas d'un coussin calorifique, la matière thermique des blocs est sélectionnée parmi celles qui, portées à la température normale d'utilisation et soumises ensuite à un chauffage, captent une quantité de chaleur importante.
Dans le cas d'un coussin isothermique, la matière thermique des blocs est sélectionnée parmi celles qui s'opposent au transfert de chaleur ou qui, portées à la température normale d'utilisation, nécessitent ensuite un apport important de chaleur pour modifier sa température. Les matières thermiques possédant, à la température normale d'utilisation, une chaleur spécifique élevée conviennent en général pour les trois types de coussins selon l'invention. Ces matières thermiques sont celles connues et utilisées en technique pour leur propriété d'accumuler des quantités importantes de chaleur et qui trouvent notamment des applications en tant qu'accumulateurs thermiques.

Dans le coussin thermique selon l'invention, les blocs peuvent être entièrement constitués par la matière thermique qui est alors nécessairement solide à la température normale d'utilisation, ainsi que dans les conditions normales de manutention du coussin thermique. En variante, les blocs peuvent, en plus de la matière thermique, comprendre une autre matière, qui ne réalise pas, à elle-seule, la fonction de la matière thermique telle que définie plus haut.
La matière thermique peut être identique pour tous les blocs ou être différente selon les blocs. Par ailleurs, chaque bloc peut comprendre une seule matière thermique ou un mélange de deux ou plusieurs matières thermiques. Dans le présent mémoire, l'expression « matière thermique » désigne indifféremment une matière thermique unique ou un mélange de matières thermiques distinctes.

Dans une forme de réalisation particulière du coussin thermique selon l'invention, les blocs comprennent des cellules à l'intérieur desquelles est disposées la matière thermique. Cette forme de réalisation du coussin thermique selon l'invention est spécialement bien adaptée au cas où la matière thermique des blocs n'est pas solide à la température normale d'utilisation ou dans les conditions normales de manutention du coussin thermique. Dans cette forme de réalisation du coussin selon l'invention, la forme extérieure des cellules doit respecter les conditions de forme exposées plus haut en ce qui concerne les blocs. Le choix du matériau des cellules est conditionné par la nécessité, pour lesdites cellules, d'être chimiquement inertes vis-à-vis de la matière thermique qu'elles contiennent et vis-à-vis de l'environnement chimique et thermique pendant leur utilisation normale. Les cellules doivent par ailleurs posséder des propriétés mécaniques compatibles avec les sollicitations mécaniques auxquelles le coussin est normalement soumis à la température normale d'utilisation ou en cours de manutention et être sensiblement indéformables (elles doivent être en un matériau rigide ou semi-rigide et de forme adaptée). Dans ce cas particulier, chaque cellule peut être entièrement isolée des cellules voisines ou, en variante, les cellules peuvent communiquer entre-elles, pour permettre au contenu fluide desdites cellules de circuler entre celles-ci. Dans le cas où la matière thermique du coussin est liquide ou gazeuse, la paroi des cellules est généralement imperméable aux liquides ou aux gaz. Les matières plastiques conviennent généralement bien pour la constitution des cellules. Des matières plastiques utilisables comprennent les polyoléfines, particulièrement les polymères et les copolymères de l'éthylène et du propylène, les polymères chlorés, particulièrement les polymères et les copolymères du chlorure de vinyle et du chlorure de vinylidène, les copolymères styrène-éthylène-butyle-styrène et le polyuréthane. Le polyéthylène, le polypropylène, le polychlorure de vinyle et les copolymères styrène-éthylène-butyle-styrène conviennent bien dans la majorité des applications. En variante, les cellules peuvent également être réalisées en métal, par exemple par emboutissage de feuilles de métal ou d'alliage malléable tels que l'aluminium et les alliages d'aluminium. Selon une autre variante, les cellules ou certaines d'entre-elles sont formées de matières composites : une partie de la cellule est en une matière et une autre partie de la cellule est réalisée en une autre matière. Selon une variante supplémentaire, une partie du réseau de cellules sont réalisées en une matière et une autre partie du réseau de cellules sont réalisées en une autre matière.

Tout procédé connu de mise en forme peut être utilisé pour fabriquer le réseau de cellules. Les procédés par moulage, emboutissage, extrusion, injection, conviennent bien.
Selon une méthode adéquate, on forme une feuille gaufrée en matière plastique ou en métal, qui présente ainsi un réseau d'alvéoles ou semi-cellules. On colle ou soude ensuite une autre feuille sur la feuille gaufrée, de manière à obturer les alvéoles de la feuille gaufrée et former les cellules. Cette autre feuille peut être une feuille unie ou une feuille gaufrée.

Dans une forme de réalisation préférée de l'invention, la matière thermique des blocs comprend une substance qui subit un changement d'état à la température normale d'utilisation. Dans cette forme de réalisation de l'invention, l'expression « changement d'état » est considérée dans son acceptation générale et désigne tout changement physique et/ou chimique de la substance, se produisant à une température sensiblement constante et caractérisé par une chaleur latente de changement d'état. Selon la substance utilisée, le changement d'état peut notamment comprendre la fusion d'un solide, la solidification d'un liquide, la vaporisation d'un liquide, la condensation d'un gaz, l'hydratation totale ou partielle d'un sel, la déshydratation totale ou partielle d'un sel, la cristallisation d'un solide amorphe ou la recristallisation d'une forme allotropique d'un cristal dans une autre forme allotropique. Dans cette forme de réalisation de l'invention, la matière thermique des blocs peut être exclusivement constituée par ladite substance qui subit un changement d'état à la température normale d'utilisation. En variante, la matière thermique des blocs peut en outre contenir d'autres substances ou matériaux qui ne subissent pas un changement d'état à la température normale d'utilisation. Par la suite, pour éviter de surcharger inutilement le texte, on supposera que c'est la matière thermique tout entière qui subit un changement d'état à la température normale d'utilisation. Il est toutefois bien entendu que, comme exposé plus haut, l'invention n'exclut pas le cas où seule une partie de la matière thermique des blocs subit un changement d'état (en l'occurrence, lorsqu'elle est composée d'un mélange de plusieurs composés chimiques dont un seul subit un changement d'état à la température normale d'utilisation ou lorsqu'on y incorpore un matériau additionnel qui ne participe pas à la fonction thermique du coussin thermique, par exemple un aimant).

Dans la forme de réalisation préférée définie ci-dessus, le changement d'état subi par la matière thermique des blocs à la température normale d'utilisation correspond à une absorption de chaleur par ladite matière dans le cas d'un coussin cryogénique et à une cession de chaleur dans le cas d'un coussin calorifique.
Dans le cas d'un coussin isothermique, le changement d'état subi par la matière thermique des blocs à la température normale d'utilisation correspond à une absorption de chaleur par ladite matière thermique lorsque ladite température normale d'utilisation (qui est aussi celle du corps auquel on destine le coussin) est inférieure à celle du milieu ambiant. Le changement d'état correspond à une cession de chaleur par la matière thermique, lorsque la température normale d'utilisation est supérieure à celle du milieu ambiant.

Pour utiliser un coussin thermique conforme à la forme de réalisation préférée qui vient d'être décrite, il convient de sélectionner convenablement l'état de la matière thermique des blocs. Dans le cas où on utilise le coussin en tant que coussin cryogénique, il est nécessaire d'amener d'abord la matière thermique des blocs dans un état qui, à la température normale d'utilisation va subir un changement d'état correspondant à une absorption de chaleur (par exemple la fusion d'un solide ou la vaporisation d'un liquide). Dans le cas où on utilise le coussin en tant que coussin calorifique, il est nécessaire d'amener d'abord la matière thermique des blocs dans un état qui, à la température normale d'utilisation va subir un changement d'état correspondant à une production de chaleur (par exemple la solidification ou la cristallisation d'un liquide ou la condensation d'un gaz). Dans le cas où on utilise le coussin en tant que coussin isothermique, dans un milieu ambiant dont la température est supérieure à la température normale d'utilisation, il est nécessaire d'amener d'abord la matière thermique des blocs dans un état qui, à la température normale d'utilisation va subir un changement d'état correspondant à une absorption de chaleur (par exemple la fusion d'un solide ou la vaporisation d'un liquide). Dans le cas où on utilise le coussin en tant que coussin isothermique, dans un milieu ambiant dont la température est inférieure à la température normale d'utilisation, il est nécessaire d'amener d'abord la matière thermique des cellules dans un état qui, à la température normale d'utilisation va subir un changement d'état correspondant à une production de chaleur (par exemple la solidification ou la cristallisation d'un liquide ou la condensation d'un gaz. Toutes autres choses étant égales par ailleurs, dans la forme de réalisation préférée qui vient d'être décrite, les meilleurs résultats sont obtenus avec des matières thermiques qui, à la température normale d'utilisation, possèdent une chaleur latente de changement d'état élevée.

Dans la forme de réalisation préférée qui vient d'être décrite, la matière thermique des blocs, subissant un changement d'état à la température normale d'utilisation peut être un corps pur. En variante, elle peut être une composition chimique qui est congruente à ladite température normale d'utilisation, de manière que le changement d'état s'opère à une température sensiblement constante.

Dans la forme de réalisation préférée décrite ci-dessus et ses variantes de réalisation, le choix de la matière thermique des blocs est conditionné par la température normale d'utilisation du coussin. Celle-ci est elle-même conditionnée par l'application à laquelle on destine le coussin thermique. L'eau et les solutions aqueuses conviennent généralement bien dans le cas particulier d'un coussin cryogénique destiné à des applications thérapeutiques. L'eau pure convient bien dans le cas d'applications où la température normale d'utilisation du coussin est voisine de 273 K (0 °C). Pour des applications où la température normale d'utilisation est inférieure à 273 K, on préconise des solutions aqueuses dans lesquelles le corps dissous et sa concentration sont sélectionnés en fonction de la température normale d'utilisation du coussin cryogénique. On recommande de sélectionner le corps dissous et sa concentration de manière à éviter une précipitation partielle ou totale dudit corps dissous à la température normale d'utilisation du coussin. Des exemples de corps dissous comprennent le chlorure de sodium, le chlorure de calcium, le carbonate de sodium, le propylène glycol, la glycérine, l'alcool éthylique et l'alcool propylique.

Comme exposé plus haut, lorsque la matière thermique des blocs est liquide, celle-ci est enfermée dans des cellules dont la paroi est généralement imperméable aux liquides. Lorsqu'on utilise une matière thermique liquide qui subit une vaporisation à la température normale d'utilisation, il peut se révéler avantageux d'utiliser, pour les cellules, une paroi à perméabilité orientée ou une paroi qui est imperméable à la phase liquide de la matière thermique, mais perméable à sa phase gazeuse (par exemple PU imper-respirant). L'invention n'est toutefois pas limitée à cette forme de réalisation et couvre également le cas où la paroi des cellules est imperméable à la phase liquide et à la phase gazeuse de la matière thermique des cellules.

Dans le coussin thermique selon l'invention, la matière thermique des blocs constitue l'élément actif principal du coussin et lui confère ses propriétés thermiques (propriétés cryogéniques, calorifiques ou isothermiques, selon sa destination). La matière thermique des interstices a pour fonction d'accroître l'efficacité du coussin, en augmentant sa surface active et son volume actif. La matière thermique des interstices doit posséder des propriétés thermiques analogues à celles énoncées plus haut pour la matière thermique des blocs. Ses propriétés thermiques doivent dès lors être adaptées à la destination du coussin, à la température normale d'utilisation de celui-ci, à la température du corps auquel on destine le coussin et à la température ambiante. Pour ce qui concerne les propriétés thermiques de la matière thermique des interstices, on peut par conséquent répéter ce qui a été exposé plus haut pour la matière thermique des blocs.

La matière thermique des interstices doit par ailleurs être déformable. Cette propriété additionnelle de la matière thermique des interstices est nécessaire pour permettre le mouvement des blocs articulés et la déformation du coussin. Le choix de la matière thermique des interstices et/ou son mode de mise en oeuvre sont dès lors conditionnés par les paramètres constructifs du coussin, tels que la forme des blocs, la forme des interstices séparant les blocs, les articulations des blocs et la position de ces articulations dans le réseau de blocs.

Dans une forme de réalisation particulière du coussin thermique selon l'invention, la matière thermique des interstices est liquide ou gazeuse à la température normale d'utilisation. Dans cette forme de réalisation de l'invention, les interstices doivent être obturés pour y retenir la matière thermique. L'organe d'obturation utilisé ne doit pas faire obstacle au mouvement des blocs sur leurs articulations. L'organe d'obturation peut par exemple comprendre une membrane souple et avantageusement élastique. Des informations complémentaires concernant l'organe d'obturation des interstices seront fournies plus loin

Dans une autre forme de réalisation du coussin thermique selon l'invention, la matière thermique des interstices comprend un corps solide élastique. Dans cette forme de réalisation de l'invention, le corps solide élastique peut comprendre une mousse en une substance polymérique ou un élastomère, par exemple du caoutchouc naturel ou synthétique. Dans le cas d'une mousse, les pores de celle-ci peuvent être occupés par de l'air ou un autre gaz (azote ou argon, par exemple).

Dans une variante particulière de cette forme de réalisation de l'invention, le corps solide élastique situé dans les interstices est fixé aux blocs (par exemple par collage, soudage ou tout autre moyen approprié) et participe au moins partiellement à l'articulation du réseau de blocs.

Dans une forme de réalisation supplémentaire du coussin thermique selon l'invention, la matière thermique des interstices comprend un gel (ou fluide visqueux).

Dans le coussin thermique selon l'invention, les interstices peuvent être comblés partiellement ou totalement par la matière thermique. Dans le cas où la matière thermique n'occupe qu'une partie du volume des interstices, il suffit généralement qu'elle puisse subir une déformation sans changement de volume, lorsque l'on déforme le réseau de blocs. La situation se présente différemment dans le cas où les interstices sont comblés totalement par la matière thermique. Dans ce dernier cas, les propriétés mécaniques de la matière thermique ou la conception du réseau de blocs sont imposées par la disposition des articulations du réseau de blocs. Selon la position de ces articulations, le volume des interstices va rester invariable ou, au contraire, va changer (augmenter ou diminuer), lorsque l'on déforme le réseau de blocs. Les réseaux de blocs dont le volume des interstices reste invariable ne pose généralement pas de problème. Dans ce cas particulier, la matière thermique des interstices peut, mais ne doit pas nécessairement, être élastique. Par contre, dans les coussins où le volume des interstices change dès que l'on modifie la forme du réseau de blocs, des dispositions doivent être prises pour compenser cette variation de volume. Une première disposition consiste à sélectionner, pour les interstices, une matière thermique élastique, qui soit ainsi capable de se contracter ou de se dilater lorsqu'on déforme le réseau de blocs. Une disposition alternative consiste à permettre une expansion de la matière thermique hors des interstices ou une rétraction de celle-ci dans lesdits interstices, selon que la déformation imposée au réseau de blocs provoque une augmentation du volume des interstices ou une diminution de celui-ci.

On préfère, selon l'invention, que les interstices du réseau de blocs soient comblés totalement avec la matière thermique.

Dans le coussin thermique selon l'invention, les interstices entre les blocs peuvent être ouverts. Cette forme de réalisation de l'invention convient lorsque la matière thermique déformable des interstices est un solide déformable (par exemple une mousse) à la température normale d'utilisation et dans les conditions normales de manutention du coussin thermique.

Dans une forme de réalisation particulière du coussin thermique selon l'invention, les interstices entre les blocs sont obturés par un organe d'obturation. Cette forme de réalisation de l'invention s'impose dans le cas où la matière thermique des interstices est un gaz, un liquide ou une poudre dans les conditions normales d'utilisation et de manutention du coussin thermique. Elle convient aussi dans le cas où la matière thermique des interstices est un corps solide déformable. Dans cette forme de réalisation de l'invention, il convient de choisir un organe d'obturation qui ne fasse pas obstacle au mouvement des blocs articulés. A cet effet, l'organe d'obturation comprend avantageusement des membranes souples qui sont fixées (par exemple soudées ou collées) aux blocs. Les membranes souples sont de préférence élastiques. Dans cette forme de réalisation de l'invention, chaque interstice peut être recouvert d'une membrane individuelle. En variante, une feuille étanche et élastique recouvre une partie au moins ou la totalité du réseau de blocs et elle se substitue alors aux membranes individuelles obturant les interstices. Dans une autre variante, le réseau de blocs est enfermé dans une enveloppe étanche, souple et, de préférence, élastique. Dans cette dernière variante, l'enveloppe peut être fixée de manière appropriée aux blocs pour obturer les interstices de manière étanche lorsque la matière thermique des interstices est fluide (gaz ou liquide). Dans le cas où la matière thermique des interstices est un solide déformable (par exemple une mousse), l'enveloppe peut indifféremment être fixée à tous les blocs du réseau de blocs, à certains de ceux-ci (et pas aux autres) ou n'être fixée à aucun bloc. La fixation de l'enveloppe aux blocs peut être obtenue par tout moyen approprié. Des moyens appropriés comprennent le collage et le soudage. L'enveloppe peut être simple ou constituée de film complexe ou comporter plusieurs couches liées ou non. Elle peut être de composition distincte selon la face. Elle peut comporter des traitements et additifs qui lui confèrent des propriétés actives spécifiques selon l'utilisation.

Le coussin thermique selon l'invention présente la propriété avantageuse de combiner d'excellentes propriétés de stockage et d'échange thermique et une excellente flexibilité/élasticité qui permet de l'adapter immédiatement à la forme d'un corps sur lequel on l'applique, de telle sorte qu'il vienne épouser parfaitement la forme de ce corps et assure alors une régulation homogène de la température ou des échanges thermiques sur toute la surface couverte dudit corps. Du fait de la conception et de la structure du coussin thermique selon l'invention, l'épaisseur de celui-ci est sans effet sur sa flexibilité et son aptitude à la déformation. En d'autres termes, l'invention permet d'adapter l'épaisseur du coussin aux propriétés thermiques recherchées (à sa capacité thermique), sans nuire à sa flexibilité ni à son aptitude à s'adapter facilement à des corps de formes diverses.

Le coussin thermique selon l'invention trouve de multiples applications. Il trouve notamment une utilisation en tant que coussin isothermique (ou thermostat) pour maintenir la température de récipients tels que des bouteilles ou des flacons thermostatiques. Une telle utilisation du coussin thermique selon l'invention trouve des applications dans l'industrie pour le maintien de produits chimiques à des températures prédéterminées, ainsi que dans le secteur alimentaire pour réchauffer, refroidir ou conserver la température d'enceintes contenant des denrées alimentaires.

Le coussin thermique selon l'invention trouve également des applications en tant que coussin cryogénique, notamment dans l'industrie chimique ou pharmaceutique pour le maintien de produits chimiques à de basses températures, ainsi que dans l'industrie alimentaire pour préserver la conservation de denrées alimentaires.

Du fait de son excellente aptitude à la déformation, le coussin thermique selon l'invention est spécialement adapté aux applications médicales, para-médicales et sportives, notamment en cryothérapie. La cryothérapie est une technique médicale qui est largement utilisée, notamment pour soulager la douleur (par exemple les maux de tête, les migraines, les maux de dents, les douleurs musculaires ou les douleurs inflammatoires), pour faciliter la résorption d'hématomes ou d'oedème et la cicatrisation de plaies accidentelles ou chirurgicales.

L'invention concerne dès lors également l'utilisation du coussin selon l'invention en thérapie médicale, particulièrement en cryothérapie.

### Brève description des figures

Des particularités et détails de l'invention vont apparaître au cours de la description suivante des figures annexées, qui représentent quelques formes de réalisation particulière de l'invention.
La figure 1 est une vue schématique, en section transversale, d'une forme de réalisation particulière du coussin thermique selon l'invention ;
La figure 2 montre en perspective, avec arrachement partiel, un détail du coussin thermique de la figure 1 ;
La figure 3 montre en perspective un détail de la figure 2 ;
La figure 4 montre, en section horizontale, une autre forme de réalisation du coussin thermique selon l'invention ; et
La figure 5 montre le coussin thermique de la figure 4, en section verticale.

Les figures ne sont pas dessinées à l'échelle. Généralement, les mêmes numéros de référence désignent les mêmes éléments.

### Description détaillée de modes de réalisation particuliers

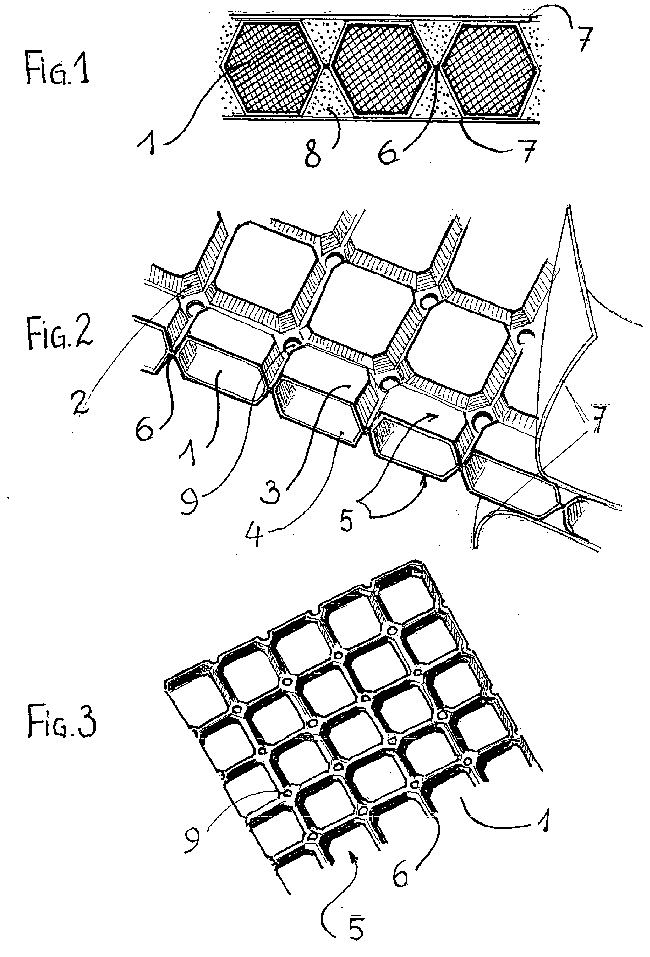

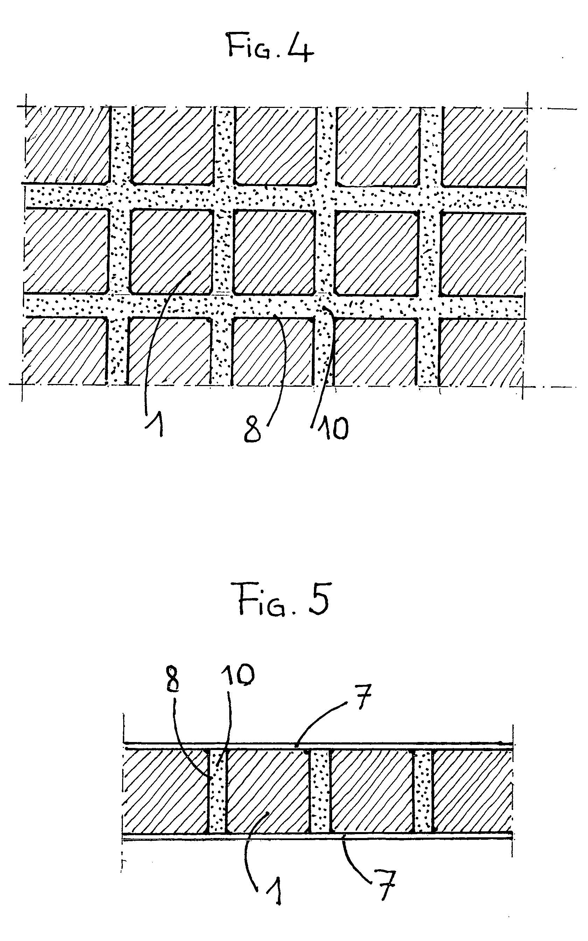

Le coussin thermique représenté aux figures 1 et 2 comprend un réseau de blocs 1, formés de cellules creuses. Les blocs ou cellules 1 ont la forme de prismes à base carrée. Chaque cellule 1 est formée de deux troncs de pyramides 3 et 4, raccordés le long de leur grande base carrée. Les arêtes latérales des deux troncs de pyramide sont chanfreinées (2). Le réseau des cellules 1 est formé de l'assemblage de deux feuilles gaufrées 5 en matière polymérique souple (par exemple en polychlorure de vinyle plastifié ou en copolymère styrène-éthylène-butyle-styrène) préalablement embouties et soudées l'une à l'autre le long de languettes 6 entre les cellules (une feuille gaufrée 5 est représentée à la figure 3). Les languettes 6 sont souples et élastiques et elles servent d'articulations entre les cellules 1. Elles sont percées d'ouvertures 9 dont la fonction apparaîtra plus loin. En pliant ou tordant l'assemblage des feuilles 5, on peut ainsi faire adopter des profils complexes au coussin de cellules, pour l'adapter à un corps approprié, par exemple une partie de l'anatomie humaine ou animale.

Le coussin thermique des figures est un coussin cryogénique, destiné à une cryothérapie. A cet effet, les cellules 1 sont remplies d'une matière thermique appropriée, dont une définition a été donnée plus haut.
La matière thermique est avantageusement une solution aqueuse dont la congélation est congruente et a lieu à une température correspondant à la température à laquelle le coussin est destiné (température normale d'utilisation). Lorsque la température normale d'utilisation est voisine de 273 K (ou 0°C), la solution aqueuse peut être remplacée par de l'eau distillée ou minéralisée.

Les interstices 8 délimités entre les cellules 1 et les languettes 6 sont comblés avec une matière thermique déformable 10(représentée à la figure 1 uniquement). La matière thermique 10 représentée à la figure 1 est un corps solide élastique, par exemple une masse en élastomère ou une mousse polymérique. Elle est de préférence choisie de manière à présenter une chaleur spécifique élevée à la température normale d'utilisation.

Une enveloppe souple et élastique 7 entoure le réseau des cellules 1 et d'interstices 8. L'enveloppe élastique 7 est par exemple un film en copolymère styrène-éthylène-butyle-styrène.

Avant d'utiliser le coussin thermique des figures 1 à 3 dans une application thérapeutique, on le maintient, par exemple dans une armoire frigorifique, à une température suffisamment basse pour congeler la solution aqueuse des cellules 1. Le coussin thermique est retiré de l'armoire frigorifique au moment de son utilisation et on peut l'appliquer immédiatement sur une partie du corps humain que l'on souhaite soumettre à une thérapie cryogénique, par exemple une main, la tête ou un bras. Grâce à la souplesse de l'enveloppe 7 et des languettes 6 et à l'élasticité de la matière thermique 10 contenue dans les interstices 8, le coussin se déforme et s'adapte parfaitement à la morphologie du corps humain.

Dans une forme de réalisation modifiée du coussin thermique des figures 1 à 3, l'enveloppe élastique 7 est soudée aux cellules 1 et mise sous tension, de telle sorte que le corps solide élastique 10 des interstices 8 soit maintenu dans un état de compression élastique dans ces interstices 8.

Lorsque le coussin thermique représenté aux figures est utilisé pour une thérapie médicale, on peut interposer une compresse entre le coussin thermique et la partie anatomique du corps humain. Toute compresse habituellement utilisée en thérapie médicale convient. Elle peut par exemple comprendre une gaze. Après avoir posé le coussin thermique sur la compresse, on le lie fermement à la partie traitée du corps, au moyen d'un bandage (adhésif ou autre), de manière qu'en se déformant, il épouse parfaitement toute la partie traitée du corps. En variante, le coussin peut être lui-même muni de parties adhésives médicales de type repositionnables. Dans ce cas particulier, le bandage est superflu.

Pour améliorer l'action thermique du coussin sur la partie anatomique traitée, on peut, avec avantage, poser un second coussin thermique sur le premier coussin thermique. Dans ce cas, seul le premier coussin thermique [celui qui est appliqué directement sur le corps humain (ou sur la compresse)] est traité dans l'armoire frigorifique, l'autre coussin thermique étant maintenu à la température ambiante. Dans cette application, le second coussin thermique peut agir comme isolant thermique. Il peut notamment faire office de bandage isolant de fixation et/ou de compression. Il peut être muni d'une poche gonflable pour l'application optimale sur des corps de forme concave.

Dans certaines formes de réalisation, le coussin peut intégrer en un article unique la fonction thermique, la fonction de bandage isolant et comprendre la compresse dont il a été question plus haut, que l'on interpose entre le coussin thermique et la partie anatomique du corps humain.

Dans une forme de réalisation modifiée du coussin thermique représenté aux figures, les languettes 6 sont percées d'ouvertures 9 (dont la fonction apparaîtra plus loin) et la matière thermique 10 des interstices 8 est à l'état liquide ou de gel à la température normale d'utilisation. Dans cette forme de réalisation du coussin, l'enveloppe 7 est fixée aux cellules 1, de manière à obturer hermétiquement les interstices 8. Les ouvertures 9 ont pour fonction de permettre une circulation de la matière thermique fluide dans le réseau d'interstices 8, lorsque le coussin subit une déformation.

La structure du coussin thermique permet éventuellement son utilisation par circulation de fluide en interstices et/ou en cellules (communicantes par canaux de remplissage/circulation).

Des réactions de mélanges thermogènes/cryogènes peuvent être utilisées pour initier la fonction thermique de manière autonome ou pour prolonger la durée d'action sans nécessiter la dépose pour recharge du coussin.

Le coussin peut comporter des éléments de contrôle et régulation de température.

Dans la forme de réalisation représentée aux figures 4 et 5, le coussin comprend un réseau de cellules 1 entre lesquelles est interposé un réseau d'une masse solide élastique 10. La masse élastique solide 10 est par exemple une masse en élastomère ou une mousse en résine polymèrique. Le réseau élastique 10 est fixé au réseau de cellules 1 par collage ou soudage. Le réseau élastique 10 sert ainsi d'articulation entre les cellules 1.

## Revendications

1. Coussin thermique comportant un réseau de blocs dont une partie au moins comprend une matière thermique, **caractérisé en ce que** les blocs (1) sont articulés et forment entre eux des interstices (8) qui sont comblés au moins partiellement avec une matière thermique déformable.

2. Coussin selon la revendication 1, **caractérisé en ce que** la forme des blocs (1) est sélectionnée parmi les formes sphérique, hémisphérique, ovoïde, annulaire, lenticulaire, conique, tronconique et polyédrique.

3. Coussin selon la revendication 2, **caractérisé en ce que** les blocs (1) sont des polyèdres à base triangulaire, carrée ou octogonale.

4. Coussin selon la revendication 3, **caractérisé en ce que** chaque bloc (1) est formé de deux troncs de pyramide (3, 4), qui sont joints le long de leur grande base et **en ce que** les blocs sont articulés sur une articulation (6) qui est disposée dans le plan géométrique de ladite grande base.

5. Coussin selon l'une quelconque des revendications 1 à 4, **caractérisé en ce que** la matière thermique déformable des interstices (8) comprend un corps solide élastique.

6. Coussin selon la revendication 5, **caractérisé en ce que** le corps solide élastique comprend une mousse ou un élastomère.

7. Coussin selon la revendication 5 ou 6, **caractérisé en ce que** le corps solide élastique des interstices (8) est fixé aux blocs (1) et sert à l'articulation de ceux-ci.

8. Coussin selon l'une quelconque des revendications 1 à 7, **caractérisé en ce que** la matière thermique déformable des interstices (8) comprend un liquide ou un gel et **en ce que** les blocs (1) sont reliés entre-eux par des articulations (6) qui sont perméables aux liquides et/ou aux gels.

9. Coussin selon la revendication 8, **caractérisé en ce que** les articulations (6) des blocs (1) comprennent des membranes flexibles et/ou élastiques et perforées (9).

10. Coussin selon l'une quelconque des revendications 1 à 9, **caractérisé en ce que** les interstices (8) sont recouverts d'une feuille souple et/ou élastique (7).

11. Coussin selon la revendication 10, **caractérisé en ce que** les interstices (8) sont comblés au moins partiellement par un corps solide élastique et **en ce que** la feuille souple et/ou élastique (7) est fixée aux blocs (1) et mise sous tension de telle sorte que le corps solide élastique des interstices soit à l'état comprimé.

12. Coussin selon la revendication 10 ou 11, **caractérisé en ce que** la feuille souple et/ou élastique (7) est étanche et enveloppe les blocs (1).

13. Coussin thermique selon l'une quelconque des revendications 1 à 12, **caractérisé en ce que** l'une au moins des matières thermiques comprend une substance qui subit un changement d'état à la température normale d'utilisation.

14. Coussin selon la revendication 13, **caractérisé en ce que** la substance susdite est un corps pur ou une composition chimique qui est congruente à la température normale d'utilisation.

15. Coussin selon l'une quelconque des revendications 1 à 14, **caractérisé en ce que** les blocs (1) comprennent des cellules contenant la matière thermique.

16. Coussin selon la revendication 15, **caractérisé en ce que**, dans le cas où la matière thermique des blocs (1) présente un état liquide ou gazeux, les cellules sont étanches audit état de la matière thermique.

17. Utilisation du coussin selon l'une quelconque des revendications 1 à 16 en thérapie médicale.

## Revendications modifiées

### Revendications modifiées conformément à la règle 86(2) CBE.

**1.** Coussin thermique comportant un réseau de blocs reliés par des articulations et séparés par des interstices, lesdits blocs comprenant une matière thermique et lesdits interstices étant comblés, au moins partiellement, avec une matière thermique déformable, **caractérisé en ce que** les articulations sont sélectionnées parmi
- les corps solides élastiques qui sont fixés aux blocs (1) et constituent alors une partie au moins de la matière thermique déformable susdite ;
- les feuilles souples et/ou élastiques (7), qui sont fixées aux blocs (1) et mise sous tension de manière à comprimer un corps solide élastique, présent dans les interstices (8), ledit corps solide élastique constituant alors une partie au moins de la matière thermique déformable ; et
- les articulations (6), qui sont perméables à un liquide et/ou un gel et/ou un gaz présent dans les interstices (8), ledit liquide et/ou gel et/ou gaz constituant alors une partie au moins de la matière thermique déformable.

**2.** Coussin selon la revendication 1, **caractérisé en ce que**, dans le cas où la matière thermique déformable des interstices (8) comprend un corps solide élastique, celui-ci comprend une mousse et/ou un élastomère.

**3.** Coussin selon la revendication 1 ou 2, **caractérisé en ce que** les articulations perméables, comprennent des membranes flexibles et/ou élastiques et perforées (9).

**4.** Coussin selon l'une quelconque des revendications 1 à 3, **caractérisé en ce qu'**une feuille souple et/ou élastique (7) enveloppe les blocs (1).

**5.** Coussin selon l'une quelconque des revendications 1 à 4, **caractérisé en ce que** la forme des blocs (1) est sélectionnée parmi les formes sphérique, hémisphérique, ovoïde, annulaire, lenticulaire, conique, tronconique et polyédrique.

**6.** Coussin selon la revendication 5, **caractérisé en ce que** les blocs (1) sont des polyèdres à base triangulaire, carrée ou octogonale.

**7.** Coussin selon la revendication 6, **caractérisé en ce que** chaque bloc (1) est formé de deux troncs de pyramide (3, 4), qui sont joints le long de leur grande base et **en ce que** les blocs sont articulés sur une articulation (6) qui est disposée dans le plan géométrique de ladite grande base.

**8.** Coussin selon l'une quelconque des revendications 1 à 7, **caractérisé en ce que** les blocs (1) comprennent des cellules contenant la matière thermique.

**9.** Coussin selon la revendication 8, **caractérisé en ce que**, dans le cas où la matière thermique des blocs (1) présente un état liquide ou gazeux, les cellules sont étanches audit état de la matière thermique.

**10.** Coussin thermique selon l'une quelconque des revendications 1 à 9, **caractérisé en ce que** l'une au moins des matières thermiques comprend une substance qui subit un changement d'état à la température normale d'utilisation.

**11.** Coussin selon la revendication 10, **caractérisé en ce que** la substance susdite est un corps pur ou une composition chimique qui est congruente à la température normale d'utilisation.
